(19) Europäisches Patentamt
European Patent Office
Office européen des brevets

(11) **EP 3 988 028 A1**

(12) **EUROPEAN PATENT APPLICATION**

(43) Date of publication:
**27.04.2022 Bulletin 2022/17**

(21) Application number: **21200591.2**

(22) Date of filing: **01.10.2021**

(51) International Patent Classification (IPC):
**A61B 8/08** (2006.01) **A61B 8/06** (2006.01)

(52) Cooperative Patent Classification (CPC):
**A61B 8/0891; A61B 8/5223;** A61B 8/06;
A61B 8/0883

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.10.2020 CN 202011158043**
**29.12.2020 PCT/CN2020/140853**
**27.08.2021 US 202117434526**

(71) Applicant: **CHISON Medical Technologies Co., Ltd. Wuxi, Jiangsu 214028 (CN)**

(72) Inventors:
• **ZHANG, Yong**
**Wuxi, 214028 (CN)**
• **LU, Kuan**
**Wuxi, 214028 (CN)**
• **FEI, Zhijiang**
**Wuxi, 214028 (CN)**
• **DAI, Xiao**
**Wuxi, 214028 (CN)**

(74) Representative: **Finnegan Europe LLP**
**1 London Bridge**
**London SE1 9BG (GB)**

(54) **PULSE WAVE VELOCITY MEASURING METHOD AND ULTRASOUND DEVICE**

(57) The disclosure relates to the technical field of image processing, in particular to a pulse wave velocity measuring method and an ultrasound device. The method includes: acquiring a target blood vessel ultrasound image of a target body, the target blood vessel ultrasound image including at least two sampling areas; based on the target blood vessel ultrasound image, acquiring a distance between the at least two sampling areas; analyzing images in the at least two sampling areas to acquire a time difference of displacement of preset points in a cardiac cycle between the at least two sampling areas; and based on the above distance and time difference, determining a pulse wave velocity of the target body. According to the disclosure, the pulse wave velocity is acquired through quantitative calculation from the target blood vessel ultrasound image, and therefore the accuracy of determining the pulse wave velocity is improved.

Acquire a target blood vessel ultrasound image of a target body — S11

Based on the target blood vessel ultrasound image, acquire a distance between at least two sampling areas — S12

Analyze images in the at least two sampling areas to acquire a time difference of displacement of preset points in a cardiac cycle between the at least two sampling areas — S13

Based on the distance between the at least two sampling areas and the time difference of displacement of the preset points in the cardiac cycle between the at least two sampling areas, determine a pulse wave velocity of the target body — S14

Fig. 1

EP 3 988 028 A1

# Description

## Field

The present disclosure relates to the technical field of image processing, in particular to a pulse wave velocity measuring method and an ultrasound device.

## Background

Pulse wave velocity (PWV) refers to the conduction velocity of a pressure wave propagating along the wall of a main artery resulting from each pulsatile blood ejection of the heart. Generally, the principle that the conduction velocity of fluctuation (namely, pulse waves) generated by blood output by the heart passing through blood vessels during arteriosclerosis is increased can be applied to measure the conduction velocity of fluctuation between two heartbeats so as to judge the elasticity degree of the blood vessels; and also, PWV may be used to estimate blood pressure and the like. Therefore, the method has great clinical significance on accurate measurement of PWV

Measurement of PWV in the prior art is generally carried out by a dedicated PWV measurement machine, the existing measurement machine is provided with four probes, one probe acts on the carotid artery and the other three probes may test PWV at three points, such as the carotid artery to the radial artery, femoral artery and dorsalis pedis artery. The working principle is that pulsation is judged according to the change track of echo of the wall of a blood vessel. During measurement, the length of a blood vessel between the carotid artery and the radial artery may be estimated by using two probes corresponding to the carotid artery and the radial artery; meanwhile, signals measured by the two probes corresponding to the carotid artery and the radial artery may be analyzed to determine a time difference between the two measurement points; and finally, the PWV is calculated by using the estimated length of the blood vessel and the time difference.

However, in the above technical solution, the length of the blood vessel is estimated by using the positions of the probes, and the time difference is acquired by analyzing the signals measured by the two probes, so that the estimated length of the blood vessel has a certain estimation error, and the time difference determined by using the signals measured by the two probes also has a certain error, so that the accuracy of the measured PWV is low.

## Summary

On that account, embodiments of the present disclosure provide a pulse wave velocity measuring method and an ultrasound device so as to solve the problem of low PWV measurement accuracy.

According to a first aspect, an embodiment of the present disclosure provides a pulse wave velocity measuring method, comprising:

acquiring a target blood vessel ultrasound image of a target body, wherein the target blood vessel ultrasound image comprises at least two sampling areas; based on the target blood vessel ultrasound image, acquiring a distance between the at least two sampling areas; analyzing images in the at least two sampling areas to acquire a time difference of displacement of preset points in a cardiac cycle between the at least two sampling areas; and based on the distance between the at least two sampling areas and the time difference of displacement of the preset point in the cardiac cycle between the at least two sampling areas, determining a pulse wave velocity of the target body.

According to the pulse wave velocity measuring method provided by the embodiment of the present disclosure, through the at least two sampling areas in the target blood vessel ultrasound image, the distance between the sampling areas and the time difference between the preset points are determined based on the sampling areas in the target blood vessel ultrasound image, that is, the pulse wave velocity of the target body is quantitatively calculated from the target blood vessel ultrasound image, and therefore, the accuracy of determining the pulse wave velocity of the target body is improved.

In combination with the first aspect, in a first implementation mode of the first aspect, there are two sampling areas, and based on the distance between the at least two sampling areas and the time difference of displacement of the preset points in the cardiac cycle between the at least two sampling areas, determining the pulse wave velocity of the target body comprises:

acquiring the distance between the two sampling areas and the time difference of displacement of the preset points in the cardiac cycle between the two sampling areas under preset measurement times; calculating a ratio of the distance between the two sampling areas to the time difference of displacement of the preset points in the cardiac cycle between the two sampling areas under each measurement to acquire target pulse wave velocities in one-to-one correspondence with the measurement times; and based on the target pulse wave velocities, determining the pulse wave velocity of the target body.

According to the pulse wave velocity measuring method provided by the embodiment of the present disclosure, under the condition that two sampling areas are arranged, the target pulse wave velocities are acquired through multiple times of measurement, and then the pulse wave velocity of the target body is determined on this basis, so that errors caused by single measurement may be avoided, and the accuracy of increasing the pulse wave velocity of the target body is further improved.

In combination with the first aspect, in a second implementation mode of the first aspect, there are at least three sampling areas, and based on the distance between the at least two sampling areas and the time difference of displacement of the preset points in the cardiac cycle between the at least two sampling areas, determining the pulse wave velocity of the target body comprises:

acquiring a distance and a time difference corresponding to each set of sampling area combination under single measurement, wherein each sampling area combination is a combination of any two sampling areas in the at least three sampling areas; calculating a ratio of the distance to the time difference corresponding to each set of sampling area combination so as to acquire target pulse wave velocities in one-to-one correspondence with the sampling area combinations; and based on the target pulse wave velocity, determining the pulse wave velocity of the target body.

According to the pulse wave velocity measuring method provided by the embodiment of the present disclosure, on the basis of arrangement of at least three sampling areas, any two sampling areas are utilized to form at least two sets of sampling area combinations, so that under the condition of single measurement, different sampling area combinations may be utilized to acquire corresponding target pulse wave velocities, and based on the target pulse wave velocities corresponding to the sampling area combinations, the pulse wave velocity of the target body is determined. According to the method, on the one hand, the accuracy of determining the pulse wave velocity of the target body is improved, on the other hand, at least two target pulse wave velocities may be acquired through one-time measurement, and the efficiency of determining the pulse wave velocity of the target body is improved.

In combination with the first implementation mode of the first aspect or the second implementation mode of the first aspect, in a third implementation mode of the first aspect, based on the target pulse wave velocities, determining the pulse wave velocity of the target body comprises:

calculating a confidence level of the target pulse wave velocities; and based on the calculated confidence level, screening the target pulse wave velocities, and determining the pulse wave velocity of the target body.

According to the pulse wave velocity measuring method provided by the embodiment of the present disclosure, the target pulse wave velocities are screened by calculating the confidence level of the target pulse wave velocity, so that the reliability of the target pulse wave velocities acquired after screening can be ensured, and further the accuracy of calculation of the pulse wave velocity of the target body is ensured.

In combination with the first aspect, in a fourth implementation mode of the first aspect, acquiring the target blood vessel ultrasound image of the target body comprises:

in response to the setting operation of a working mode, determining the working mode, wherein the working mode comprises a pulse Doppler mode or an M mode; based on the working mode, collecting a blood vessel ultrasound image of the target body; and forming at least two sampling gates or sampling lines on the blood vessel ultrasound image to acquire the target blood vessel ultrasound image.

According to the pulse wave velocity measuring method provided by the embodiment of the present disclosure, corresponding to different working modes, different sampling areas are formed on the blood vessel ultrasound image, so that the reliability of arrangement of the sampling areas can be ensured.

In combination with the fourth implementation mode of the first aspect, in a fifth implementation mode of the first aspect, forming at least two sampling gates or sampling lines on the blood vessel ultrasound image to acquire the target blood vessel ultrasound image comprises:

in response to the operation of arranging the at least two sampling gates or sampling lines on the blood vessel ultrasound image, forming at least two sampling gates or sampling lines on the blood vessel ultrasound image to acquire the target blood vessel ultrasound image.

According to the pulse wave velocity measuring method provided by the embodiment of the present disclosure, the sampling areas are manually arranged on the blood vessel ultrasound image, so that the arranged sampling area can meet the requirements of a user.

In combination with the fourth implementation mode of the first aspect, in a sixth implementation mode of the first aspect, forming at least two sampling gates or sampling lines on the blood vessel ultrasound image to acquire the target blood vessel ultrasound image comprises:

acquiring at least two preset positions on the blood vessel ultrasound image; and respectively forming the sampling gates or sampling lines at the at least two preset positions to acquire the target blood vessel ultrasound image.

According to the pulse wave velocity measuring method provided by the embodiment of the present disclosure, the sampling areas are automatically formed on the blood vessel ultrasound image, so that the efficiency of arranging the sampling areas is improved, and further, the efficiency of determining the pulse wave velocity of the target body is improved.

In combination with the first aspect, in a seventh implementation mode of the first aspect, analyzing the images in the at least two sampling areas to acquire the time difference of displacement of the preset points in the cardiac cycle between the at least two sampling areas comprises:

carrying out binarization processing on the images in the at least two sampling areas to acquire first images corresponding to the sampling areas;

extracting envelope lines in the first images, and determining positions corresponding to the preset points in the cardiac cycle; and

determining the time difference of displacement of the preset points in the cardiac cycle between the at least two sampling areas by utilizing the position of the preset point.

According to the pulse wave velocity measuring method provided by the embodiment of the present disclosure, the images in the sampling areas are subjected to binarization processing before the envelope lines are extracted, so that on the one hand, the image analysis efficiency is ensured, on the other hand, the data processing amount during subsequent envelope line extraction is reduced, and the efficiency of determining the pulse wave velocity of the target body is further improved.

In combination with the seventh implementation mode of the first aspect, in an eighth implementation mode of the first aspect, carrying out binarization processing on the images in the at least two sampling areas to acquire the first images corresponding to the sampling areas comprises:

extracting a gray scale map corresponding to the images in the at least two sampling areas;

calculating an entropy value corresponding to each gray scale in the gray scale map;

determining a gray threshold by utilizing the entropy value acquired by calculation;

screening pixel points in the gray scale map based on the gray scale threshold to acquire effective pixel points in the gray scale map; and

forming the first images by utilizing the effective pixel points.

According to the pulse wave velocity measuring method provided by the embodiment of the present disclosure, the gray scale threshold is determined by utilizing the entropy value corresponding to each gray scale in the gray scale map, then the determined gray scale threshold is utilized to screen the pixel points in the gray scale map to form the first images, wherein because the gray scale threshold is determined by utilizing the entropy value corresponding to each gray scale, and is not set artificially, the reliability of pixel point screening can be ensured, and thus the accuracy of the formed first images is improved.

In combination with the eighth implementation mode of the first aspect, in a ninth implementation mode of the first aspect, forming the first images by utilizing the effective pixel points comprises:

forming second images by utilizing the effective pixel points; and

carrying out corrosion followed by expansion on the second images to acquire the first images.

According to the pulse wave velocity measuring method provided by the embodiment of the present disclosure, corrosion and expansion are carried out on the basis of the effective pixel points, isolated points and burrs in the second images may be removed, and thus the reliability of the first images is further improved.

In combination with the first aspect, or the first implementation mode or the second implementation mode of the first aspect, or any of the fourth implementation mode to the ninth implementation mode, in a tenth implementation mode of the first aspect, the method further comprises:
determining the blood pressure of the target body by utilizing the pulse wave velocity of the target body.

According to the pulse wave velocity measuring method provided by the embodiment of the present disclosure, the blood pressure of the target body is determined on the basis of the pulse wave velocity of the target body, and the accuracy of determining the blood pressure of the target body may be ensured.

According to a second aspect, an embodiment of the present disclosure also provides a pulse wave velocity measuring device, comprising:

an acquiring module, used for acquiring a target blood vessel ultrasound image of a target body, wherein the target blood vessel ultrasound image comprises at least two sampling areas;

a distance determining module, used for acquiring a distance between the at least two sampling areas based on the target blood vessel ultrasound image;

a time difference determining module, used for analyzing images in the at least two sampling areas to acquire a time difference of displacement of preset points in a cardiac cycle between the at least two sampling areas; and

a pulse wave velocity determining module, used for determining a pulse wave velocity of the target body based on the distance between the at least two sampling areas and the time difference of displacement of the preset points in the cardiac cycle between the at least two sampling areas.

According to the pulse wave velocity measuring device provided by the embodiment of the present disclosure, at least two sampling areas are formed in the target blood vessel ultrasound image, and the distance between the sampling areas and the time difference between the preset points are determined based on the sampling areas in the target blood vessel ultrasound image subsequently, namely, the pulse wave velocity of the target body is quantitatively calculated from the target blood vessel ultrasound image, so that the accuracy of determining the pulse wave velocity of the target body is improved.

According to a third aspect, an embodiment of the present disclosure provides an ultrasound device, comprising: a memory and a processor, wherein the memory and the processor are in communication connection with each other, computer instructions are stored in the memory, the processor executes the computer instructions so as to execute the pulse wave velocity measuring method of the first aspect or any one of the implementation modes of the first aspect.

According to a fourth aspect, an embodiment of the present disclosure provides a computer-readable storage medium storing computer instructions for enabling a computer to execute the pulse wave velocity measuring method of the first aspect or any one of implementation modes of the first aspect.

## Brief Description of the Drawings

In order to more clearly illustrate the specific implementation modes of the present disclosure or the technical solutions in the prior art, the drawings used in the implementation modes or descriptions in the prior art will be briefly described below, and it is obvious that the drawings in the following description are some embodiments of the present disclosure, and other drawings can be acquired by those skilled in the art without creative efforts.

Fig. 1 is a flow chart of a pulse wave velocity measuring method according to an embodiment of the present disclosure;
Fig. 2 is a flow chart of a pulse wave velocity measuring method according to an embodiment of the present disclosure;
Fig. 3 is a flow chart of a pulse wave velocity measuring method according to an embodiment of the present disclosure;
Fig. 4 is a flow chart of a pulse wave velocity measuring method according to an embodiment of the present disclosure;
Fig. 5 is a structural block diagram of a pulse wave velocity measuring device according to an embodiment of the present disclosure; and
Fig. 6 is a structural schematic diagram of hardware of an ultrasound device provided by an embodiment of the present disclosure.

## Detailed Description of the Embodiments

In order to make the objects, technical solutions and advantages of the embodiments of the present disclosure clearer, the technical solutions in the embodiments of the present disclosure will be clearly and completely described below in combination with the drawings in the embodiments of the present disclosure, and it is obvious that the described embodiments are some, but not all embodiments of the present disclosure. All other embodiments, which can be acquired by those skilled in the art without making creative efforts based on the embodiments in the present disclosure, belong to the scope of protection of the present disclosure.

It should be noted that a pulse wave velocity measuring method provided by an embodiment of the present disclosure may be applied to any electronic equipment with an image processing function, such as a computer, a mobile phone and an ultrasound device. In the following embodiments, the ultrasound device is described in detail as an example.

According to an embodiment of the present disclosure, an embodiment of a pulse wave velocity measuring method is provided, it needs to be noted that steps shown in the flow chart of the figure may be executed in a computer system such as a set of computer executable instructions, and that, although a logical sequence is shown in the flow chart, in some cases, the steps shown or described may be executed in a sequence different from the sequence herein.

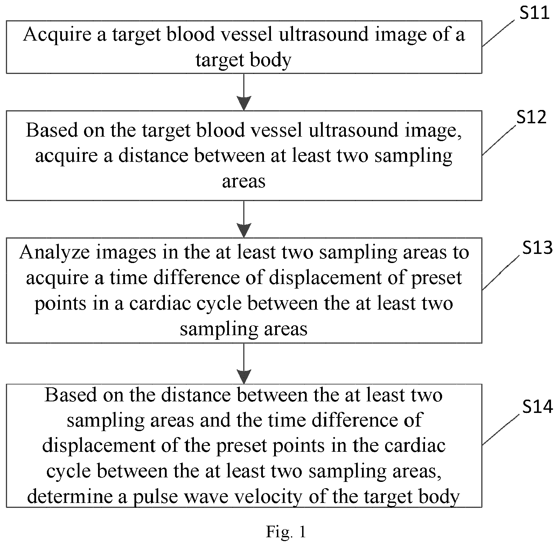

In the embodiment, a pulse wave velocity measuring method is provided, and can be used for an ultrasound device, Fig. 1 is a flow chart of a pulse wave velocity measuring method according to an embodiment of the present disclosure, as shown in Fig. 1, the flow comprises the following steps.

S11, a target blood vessel ultrasound image of a target body is acquired.

The target blood vessel ultrasound image comprises at least two sampling areas.

The target blood vessel ultrasound image may be a real-time blood vessel ultrasound image of the target body, and may also be a historical blood vessel ultrasound image of the target body or the like, and the source of the target blood vessel ultrasound image is not limited in any way herein.

For the at least two sampling areas in the target blood vessel ultrasound image, at least two sampling areas may be formed in the blood vessel ultrasound image after the blood vessel ultrasound image is acquired; or after the ultrasound device is started, at least two sampling areas are formed on a display interface of the ultrasound device, and then the blood vessel ultrasound image of the target body is collected, so that at least two sampling areas are formed in the blood vessel ultrasound image, and thus the target blood vessel ultrasound image is acquired.

The sampling areas may be automatically formed or may also be formed in an interactive manner. The specific mode of forming the sampling areas is not limited, and corresponding setting may be carried out according to actual conditions.

A number of the sampling areas formed in the target blood vessel ultrasound image may be two, three or four and the like, the specific set number may be correspondingly set according to requirements, and it only needs to guarantee that the number of the sampling areas formed in the target blood vessel ultrasound image is at least two. The sampling areas may also be regarded as sampling areas in the target blood vessel ultrasound image, and may be sampling gates, sampling lines and the like.

S12, based on the target blood vessel ultrasound image, a distance between the at least two sampling areas is acquired.

After the ultrasound device acquires a target blood vessel ultrasound image with at least two sampling areas, the distance between any two sampling areas in the target blood vessel ultrasound image may be determined as the positions of the sampling areas are fixed and known.

For example, there are two sampling areas, namely a sampling area A and a sampling area B, in the target blood vessel ultrasound image, and after the sampling area A and the sampling area B are determined, a distance between the sampling area A and the sampling area B may be acquired.

There are three sampling areas, namely a sampling area A, a sampling area B and a sampling area C, in the target blood vessel ultrasound image, and after the sampling area A, the sampling area B and the sampling area C are determined, a distance between the sampling area A and the sampling area B, a distance between the sampling area A and the sampling area C and a distance between the sampling area B and the sampling area C may be acquired. It should be noted that the above is only the condition that three distances may be acquired by utilizing the three sampling areas, and which distance or which distances to be specifically used in the subsequent treatment process may be correspondingly selected according to actual conditions, and no limitation is made herein.

The step will specifically be described in detail below.

S13, images in the at least two sampling areas are analyzed to acquire a time difference of displacement of preset points in a cardiac cycle between the at least two sampling areas.

After the ultrasound device forms the sampling areas, images in the sampling areas are analyzed and processed, and positions corresponding to the preset points in a cardiac cycle are determined in each sampling area. The preset point may be a starting point, an end point, or other characteristic point of the cardiac cycle, and the like, which is not limited in any way herein.

Specifically, before the images in the sampling areas are analyzed, the cardiac cycle of the target body may be determined in the target blood vessel ultrasound image, wherein a manner of determining the cardiac cycle is not limited herein, for example, a cardiac cycle detection model may be utilized, also, features corresponding to systolic and/or diastolic phases in the target blood vessel ultrasound image may be utilized, or the like.

After determining the cardiac cycle in the target blood vessel ultrasound image, the ultrasound device may determine positions corresponding to the preset points in the cardiac cycle in the sampling areas, for example, a position of a starting point of the cardiac cycle, a position of an end point of the cardiac cycle, or the like may be determined in each sampling area. After the ultrasound device determines the position of the starting point of the cardiac cycle in each sampling area, the time difference of displacement of the starting points of the cardiac cycle between any two sampling areas may be determined by carrying out spectrum analysis on images in the sampling areas, and the determined time difference is the motion time of the pulse wave between the two sampling areas.

Continuing to use the above example, there are two sampling areas, namely the sampling area A and the sampling area B, in the target blood vessel ultrasound image, the ultrasound device determines the starting point of the cardiac cycle in the sampling area A and determines the starting point of the cardiac cycle in the sampling area B, and then the ultrasound device may acquire a time difference between the two starting points by utilizing the two determined starting points.

There are three sampling areas, namely the sampling area A, the sampling area B and the sampling area C, in the target blood vessel ultrasound image, and the ultrasound device determines the starting points of the cardiac cycle in the sampling area A, the sampling area B and the sampling area C respectively, and a time difference of displacement of the starting points of the cardiac cycle between the sampling area A and the sampling area B, a time difference of displacement of the starting points of the cardiac cycle between the sampling area A and the sampling area C and a time difference of displacement of the starting points of the cardiac cycle between the sampling area B and the sampling area C may be acquired.

S14, based on the distance between the at least two sampling areas and the time difference of displacement of the preset points in the cardiac cycle between the at least two sampling areas, a pulse wave velocity of the target body is determined.

The ultrasound device acquires the distance between the at least two sampling areas in the step S12 and acquires the time difference of displacement of the preset points in the cardiac cycle between the at least two sampling areas in the step S13, and then the ultrasound device may acquire the pulse wave velocity of the target body by calculating a ratio of the distance to the time difference.

Continuing to use the above example, the sampling area A and the sampling area B are arranged in the target blood vessel ultrasound image, the distance $\Delta d$ between the sampling area A and the sampling area B and the time difference of displacement of the starting points of the cardiac cycle between the sampling area A and the sampling area B are acquired, the ratio of the distance $\Delta d$ to the time difference $\Delta t$ is calculated, and the pulse wave velocity of the target body may be acquired.

Further optionally, the ultrasound device may also carry out multiple times of measurements on the sampling area A and the sampling area B, and determine the pulse wave velocity of the target body by utilizing the results of the multiple times of measurements.

When the sampling area A, the sampling area B and the sampling area C are arranged in the target blood vessel ultrasound image, the distance $\Delta d_1$ between the sampling area A and the sampling area B, the distance $\Delta d_2$ between the sampling area A and the sampling area C and the distance $\Delta d_3$ between the sampling area B and the sampling area C as well as the time difference $\Delta t_1$ of displacement of the starting points of the cardiac cycle between the sampling area A and the sampling area B, the time difference $\Delta t_2$ of displacement of the starting points of the cardiac cycle between the sampling area A and the sampling area C, and the time difference $\Delta t_3$ of displacement of the starting points of the cardiac cycle between the sampling area B and the sampling area C are acquired. The ultrasound device may determine the pulse wave velocity of the target body by directly utilizing $\Delta d_1$ and $\Delta t_1$, or $\Delta d_2$ and $\Delta t_2$, or $\Delta d_3$ and $\Delta t_3$.

Optionally, the ultrasound device may also utilize the three sets of distances and time differences to determine the pulse wave velocity of the target body.

The step will specifically be described in detail below.

According to the pulse wave velocity measuring method provided by the embodiment, at least two sampling areas are formed in the target blood vessel ultrasound image, and the distance between the sampling areas and the time difference between the preset points are determined based on the sampling areas in the target blood vessel ultrasound image subsequently, namely, the pulse wave velocity of the target body is quantitatively calculated from the target blood vessel ultrasound image, so that the accuracy of determining the pulse wave velocity of the target body is improved.

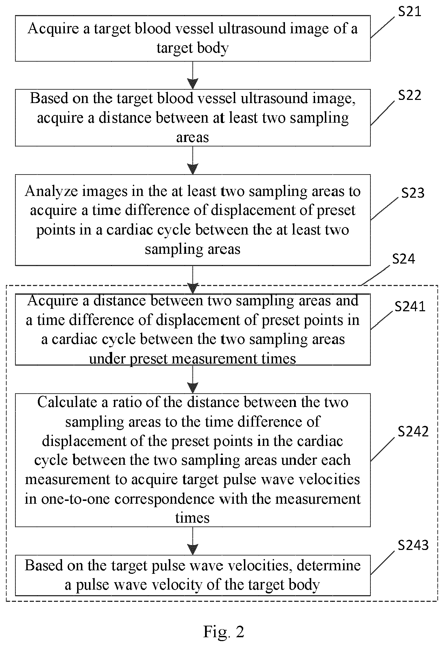

In an embodiment, a pulse wave velocity measuring method is provided, may be used in electronic equipment such as an ultrasound device, and Fig. 2 is a flow chart of a pulse wave velocity measuring method according to an embodiment of the present disclosure, as shown in Fig. 2, the flow comprises the following steps.

S21, a target blood vessel ultrasound image of a target body is acquired.

The target blood vessel ultrasound image comprises at least two sampling areas.

Referring to S11 of the embodiment as shown in Fig. 1 for details, repeated description is omitted herein.

S22, based on the target blood vessel ultrasound image, a distance between the at least two sampling areas is acquired.

Referring to S12 of the embodiment as shown in Fig. 1 for details, repeated description is omitted herein.

S23, images in the at least two sampling areas are analyzed to acquire a time difference of displacement of preset points in a cardiac cycle between the at least two sampling areas.

Referring to S13 of the embodiment as shown in Fig. 1 for details, repeated description is omitted herein.

S24, based on the distance between the at least two sampling areas and the time difference of displacement of the preset points in the cardiac cycle between the at least two sampling areas, a pulse wave velocity of the target body is determined.

When there are two sampling areas, the above S24 comprises the following steps.

S241, a distance between the two sampling areas and a time difference of displacement of preset points in the cardiac cycle between the two sampling areas under preset measurement times are acquired.

The ultrasound device may carry out multiple times of measurements on the distance and the time difference for the two sampling areas and record the distance and the time difference corresponding to each measurement.

Continuing to use the above example, the sampling area A and the sampling area B are arranged in the target blood vessel ultrasound image, and the ultrasound device carries out three times of measurements on the sampling area A and the sampling area B, the result of each measurement are shown as follows:

in the first measurement, the distance between the sampling area A and the sampling area B is $\Delta d_1$, and the time difference is $\Delta t_1$;
in the second measurement, the distance between the sampling area A and the sampling area B is $\Delta d_2$, and the time difference is $\Delta t_2$; and
in the third measurement, the distance between the sampling area A and the sampling area B is $\Delta d_3$, and the time difference is $\Delta t_3$.

S242, a ratio of the distance between the two sampling areas to the time difference of displacement of the preset points in the cardiac cycle between the two sampling areas under each measurement is calculated, and target pulse wave velocities in one-to-one correspondence with the measurement times are acquired.

The ultrasound device calculates the target pulse wave velocity by utilizing the distance and the time difference acquired by each measurement.

Continuing to use the above example, in a first test, the target pulse wave velocity 1 is $\dfrac{\Delta d_1}{\Delta t_1}$ ;

in a second test, the target pulse wave velocity 2 is $\dfrac{\Delta d_2}{\Delta t_2}$ ; and

in a third test, the target pulse wave velocity 3 is $\dfrac{\Delta d_3}{\Delta t_3}$ .

S243, based on the target pulse wave velocities, the pulse wave velocity of the target body is determined.

The ultrasound device may calculate an average value of all the target pulse wave velocities after determining the target pulse wave velocity corresponding to each measurement, and the calculated average value may be used as the pulse wave velocity of the target body; and alternatively, the pulse wave velocity of the target body may be determined by adopting the following manner. Specifically, the above S243 may comprise the following steps.

1) A confidence level of the target pulse wave velocities is calculated.

The ultrasound device may screen target pulse waves by calculating the confidence level of the target pulse wave velocities after the target pulse wave velocities corresponding to the various measurements are acquired, wherein the confidence level may be calculated by cross-correlation coefficients, or the distribution law of the target pulse wave velocities may be counted to determine the confidence level of the target pulse wave velocities.

2) The pulse wave velocity of the target body is determined by screening the target pulse wave velocities based on the calculated confidence level.

After the ultrasound device calculates the confidence level, the calculated confidence level is compared with a confidence level threshold to screen the target pulse wave velocities to acquire a target pulse velocity set P, the pulse wave velocity PWV of the target body may be calculated by using the following formula:

$$PWV = \frac{1}{n} \times \sum_{p \in P} p$$

wherein n is a number of target pulse velocities in the target pulse wave velocity set P.

The target pulse wave velocities are screened by calculating the confidence level of the target pulse wave velocities, the reliability of the target pulse wave velocities acquired after screening can be ensured, and further, the accuracy of calculation of the pulse wave velocity of the target body is ensured.

In some optional implementation modes of the embodiment, at least three sampling areas are arranged in the target blood vessel ultrasound image, and the above S24 may comprise the following steps.

(1) Distances and time differences corresponding to various sampling area combinations under single measurement are acquired.

Each sampling area combination is a combination of any two sampling areas of at least three sampling areas.

Continuing to use the above example, the sampling area A, the sampling area B and the sampling area C are arranged in the target blood vessel ultrasound image, three sets of sampling area combinations may be formed, including the sampling area A and the sampling area B, the sampling area A and the sampling area C as well as the sampling area B and the sampling area C, respectively.

The distances and time differences corresponding to the various sampling area combinations may be acquired respectively by carrying out one-time measurement on the various sampling area combinations respectively by the ultrasound device.

For example, in the sampling area combination 1 (namely the sampling area A and the sampling area B): the distance is $\Delta d_1$, and the time difference is $\Delta t_1$;

in the sampling area combination 2 (namely the sampling area A and the sampling area C): the distance is $\Delta d_2$, and the time difference is $\Delta t_2$; and
in the sampling area combination 3 (namely the sampling area B and the sampling area C): the distance is $\Delta d_3$, and the time difference is $\Delta t_3$.

(2) Ratios of the distances to the time differences corresponding to the various sets of sampling area combinations are calculated to acquire target pulse wave velocities in one-to-one correspondence with the sampling area combinations.

Corresponding to each set of sampling area combination, the ultrasound device acquires target pulse wave velocities in one-to-one correspondence with the sampling area combinations respectively by calculating the ratios of the distances to the time differences. As described above, the ultrasound device may acquire the target pulse wave velocities corresponding to the three sets of sampling area combinations by single measurement.

(3) The pulse wave velocity of the target body is determined based on the target pulse wave velocities.

The step can be described in detail with reference to the above S243, and will not be described in detail.

At least two sets of sampling area combinations are formed by utilizing any two sampling areas on the basis of arranging at least three sampling areas, and thus in the case of single measurement, the different sampling area combinations are utilized to acquire the corresponding target pulse wave velocities, and the pulse wave velocity of the target body is determined on the basis of the target pulse wave velocities corresponding to the various sampling area combinations. By the method, on the one hand, the accuracy of determining the pulse wave velocity of the target body is improved, on the other hand, at least two target pulse wave velocities may be acquired through one-time measurement, and the efficiency of determining the pulse wave velocity of the target body is improved.

According to the pulse wave velocity measuring method provided by the embodiment, under the condition that two sampling areas are arranged, the target pulse wave velocities are acquired through multiple times of measurements, the pulse wave velocity of the target body is determined on this basis, so that errors caused by single measurement may be avoided, and the accuracy of determining the pulse wave velocity of the target body is further improved.

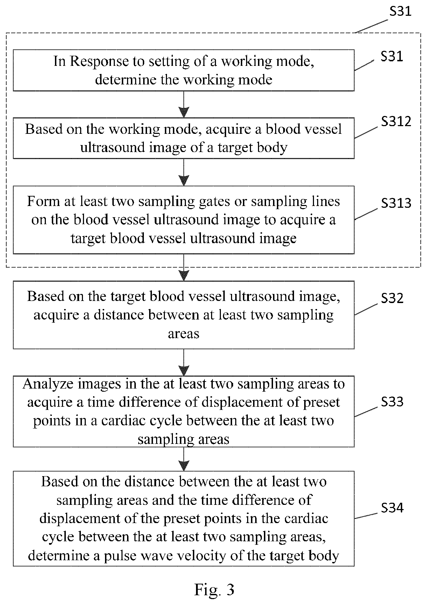

In an embodiment, a pulse wave velocity measuring method is provided, and may be used in electronic equipment, such as an ultrasound device, Fig. 3 is a flow chart of a pulse wave velocity measuring method according to an embodiment of the present disclosure, as shown in Fig. 3, the flow comprises the following steps.

S31, A target blood vessel ultrasound image of a target body is acquired.

The target blood vessel ultrasound image comprises at least two sampling areas.

Specifically, the aboveS31 comprises the following steps.

S311, in response to setting of a working mode, the working mode is determined.

The working mode comprises a pulse Doppler mode or an M mode.

When a user uses the ultrasound device to sample the target body, the working mode of the ultrasound device needs to be set, for example, the mode of the ultrasound device may be set to be a Doppler mode or an M mode. When the user sets the working mode of the ultrasound device, the ultrasound device responds to the setting operation of the user to set the working mode to be a corresponding mode, so that the working mode of the ultrasound device is determined.

S3212, the blood vessel ultrasound image of the target body is acquired based on the working mode.

After the working mode is determined by the ultrasound device, the blood vessel ultrasound image of the target body may be collected under the working mode.

S313, At least two sampling gates or sampling lines are formed on the blood vessel ultrasound image to acquire the target blood vessel ultrasound image.

When the working mode of the ultrasound device is the Doppler mode, the sampling areas formed on the blood vessel ultrasound image are sampling gates;

and when the working mode of the ultrasound device is the M mode, the sampling areas formed on the blood vessel ultrasound image are sampling lines.

The sampling areas may be formed automatically or may also be formed manually. Automatic formation of the sampling areas and manual formation of the sampling areas will be described in detail below, respectively.

(1) Automatic formation of sampling areas

1.1) At least two preset positions on the blood vessel ultrasound image are acquired.

The preset positions may be specified points in the blood vessel ultrasound image, and may also be two boundary positions of the blood vessel ultrasound image on a display interface of the ultrasound device. A number of the preset positions and specific positions are not limited in any way herein, and specifically, corresponding setting may be carried out according to actual conditions.

1.2) Sampling gates or sampling lines are respectively formed at the at least two preset positions to acquire the target blood vessel ultrasound image.

After the ultrasound device acquires the preset positions, the sampling gates or sampling lines may be formed at the determined preset position based on the working mode of the ultrasound device. After the sampling gates or sampling lines are determined, a distance between the sampling gates or sampling lines may be acquired.

Because the longer the time between the sampling areas is, the higher the calculation accuracy of the pulse wave velocity is, the two sampling gates are arranged at the boundary of the blood vessel ultrasound image on the display interface, and the accuracy of determining the pulse wave velocity of the target body may be improved.

The sampling areas are automatically formed on the blood vessel ultrasound image, so that the efficiency of arranging the sampling areas is improved, and further, the efficiency of determining the pulse wave velocity of the target body is improved.

(2) Manual formation of sampling areas

In response to the operation of arranging at least two sampling gates or sampling lines on the blood vessel ultrasound image, at least two sampling gates or sampling lines are formed on the blood vessel ultrasound image to acquire a target blood vessel ultrasound image.

A user carries out operation of arranging the sampling gates or sampling lines on the blood vessel ultrasound image, and the ultrasound device may respond to the operation of the user. After the ultrasound device responds to the operation of the user, at least two sampling gates or sampling lines are formed on the blood vessel ultrasound image, so that the target blood vessel ultrasound image is acquired.

The sampling areas are manually arranged on the blood vessel ultrasound image, so that the arranged sampling area can meet the requirements of the user.

S32, based on the target blood vessel ultrasound image, a distance between the at least two sampling areas is acquired.

Referring to S22 of the embodiment as shown in Fig. 2 for details, repeated description is omitted herein.

S33, images in the at least two sampling areas are analyzed to acquire a time difference of displacement of preset points in a cardiac cycle between the at least two sampling areas.

Referring to S23 of the embodiment as shown in Fig. 2 for details, repeated description is omitted herein.

S34, a pulse wave velocity of the target body is determined based on the distance between the at least two sampling areas and the time difference of displacement of the preset points in the cardiac cycle between the at least two sampling areas.

Please refer to S24 of the embodiment as shown in Fig. 2 for details, repeated description is omitted herein.

The pulse wave velocity measuring method provided by the embodiment corresponds to different working modes, and the sampling areas on the target blood vessel ultrasound image are different, so that the reliability of arrangement of the sampling areas can be ensured.

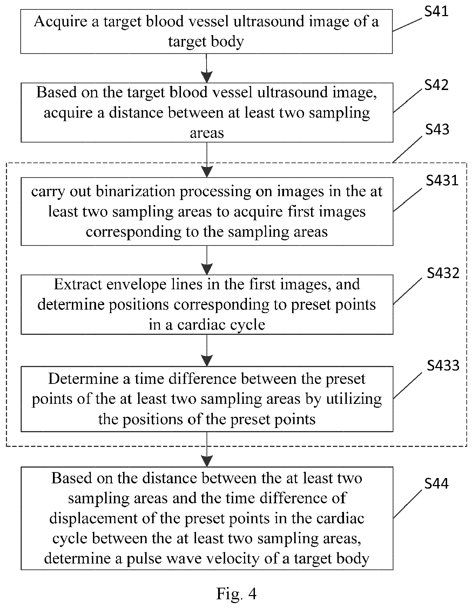

In an embodiment, a pulse wave velocity measuring method is provided, and may be used in ultrasound device, Fig. 4 is a flow chart of a pulse wave velocity measuring method according to an embodiment of the present disclosure, and as shown in Fig. 4, the flow comprises the following steps.

S41, a target blood vessel ultrasound image of a target body is acquired.

The target blood vessel ultrasound image comprises at least two sampling areas.

Referring to S11 of the embodiment as shown in Fig. 1 for details, repeated description is omitted herein.

S42, based on the target blood vessel ultrasound image, a distance between the at least two sampling areas is acquired.

Referring to S32 of the embodiment as shown in Fig. 3 for details, repeated description is omitted herein.

S43, images in the at least two sampling areas are analyzed to acquire a time difference of displacement of preset points in a cardiac cycle between the at least two sampling areas.

Specifically, the above S43 comprises the following steps.

S431, the images in the at least two sampling areas are subjected to binarization processing to acquire first images corresponding to the sampling areas.

The binarization processing may be carried out by comparing gray scale values of pixel points of the images in the sampling areas with a preset gray scale value to acquire the first images; and the binarization processing may also be carried out by other manners, which is not limited herein.

In some optional implementation modes of the embodiment, the above S431 may comprise the following steps.

(1) A gray scale map corresponding to the images in the at least two sampling areas is extracted.

After the ultrasound device extracts the images in the sampling areas, if the extracted images are not the grey scale map, the extracted images are converted into the grey scale map. A range of the gray scale value of each pixel point in the gray scale map is [0, L-1].

(2) An entropy value corresponding to each gray scale in the gray scale map is calculated.

The entropy value $E(t)$ corresponding to each gray scale in the gray scale map may be calculated by using the following formula:

$$E(t) = \lg p_t(1 - p_t) + \frac{H_t}{p_t} + \frac{H_{L-1} - H_t}{1 - p_t}$$

$$p_t = \sum_{i=0}^{t} p_i$$

$$H_t = -\sum_{i=0}^{t} p_i \lg p_i$$

$$H_{L-1} = -\sum_{i=0}^{L-1} p_i \lg p_i$$

wherein $p_i$ is a probability of occurrence of a gray scale i.

(3) A gray threshold is determined by utilizing the entropy value acquired by calculation.

After the entropy value corresponding to each gray scale is calculated, the ultrasound device may determine a maximum entropy value among all the entropy values, and determine the gray scale corresponding to the maximum value of E(t) as the gray scale threshold $I_t$.

The gray scale threshold is determined by utilizing the entropy value corresponding to each gray scale in the gray scale map, the pixel points in the gray scale map are screened by utilizing the determined gray scale threshold to form the first images, wherein as the gray scale threshold is determined by utilizing the entropy value corresponding to each gray scale instead of being manually set, the reliability of pixel point screening can be ensured, and thus the accuracy of the formed first image is improved.

(4) The pixel points in the gray scale map are screened based on the gray scale threshold to acquire effective pixel points in the gray scale map.

The ultrasound device sequentially compares the gray scale of each pixel point with the gray scale threshold $I_t$, and the pixel points with the gray scales larger than the gray scale threshold are determined to be the effective pixel points in the gray scale map.

(5) The first images are formed by utilizing the effective pixel points.

The ultrasound device may directly utilize the effective pixel points to form the first images, and may also process the effective pixel points to subsequently form the first images.

As an optional implementation mode of the embodiment, the above step (5) may comprise the following steps.

5.1) Second images are formed by utilizing the effective pixel points.

5.2) Corrosion followed by expansion are carried out on the second images to acquire the first image.

In order to remove isolated points and burrs, the images subjected to binarization need to be subjected to an opening operation of corrosion followed by expansion. The algorithm formula is as follows:

$$XOS = (X \ominus S_e) \oplus S_d$$

wherein $X$ is the second image, $S_e$ is a structural element for corrosion, and $S_d$ is a structural element for expansion.

S432, envelope lines in the first images are extracted to determine positions corresponding to the preset points in the cardiac cycle are determined.

After the ultrasound device forms the first images, the envelope lines in the first images are extracted, and therefore, the positions corresponding to the preset points in the cardiac cycle are determined.

S433, the time difference between the preset points corresponding to the at least two sampling areas is determined by utilizing the positions of the preset points.

Because a change of the ultrasound image of the target body along with time has been reflected in the target blood vessel ultrasound image, the time difference between the preset points may be determined by utilizing the determined positions of the preset points.

S44, a pulse wave velocity of the target body is determined based on the distance between the at least two sampling areas and the time difference of displacement of the preset points in the cardiac cycle between the at least two sampling areas.

Referring to S24 of the embodiment as shown in Fig. 2 for details, repeated description is omitted herein.

According to the pulse wave velocity measuring method provided by the embodiment, the images in the sampling areas are subjected to binarization processing before the envelope lines are extracted, so that on the one hand, the image analysis efficiency is ensured, on the other hand, the data processing amount during subsequent envelope line extraction is reduced, and further, the efficiency of determining the pulse wave velocity of the target body is improved.

In an optional implementation mode of the embodiment, the above pulse wave velocity measuring method may further comprise the step that the blood pressure of the target body is determined by utilizing the pulse wave velocity of the target body.

For example, a mathematical model of the pulse wave velocity and the blood pressure may be established, and the blood pressure of the target body and the like may be determined by utilizing the model and the measured pulse wave velocity. The specific mode for determining the blood pressure of the target body by utilizing the pulse wave velocity of the target body is not limited in any way, and the modes for determining the blood pressure by using the pulse wave velocity measured by the pulse wave velocity measuring method provided by the present disclosure belongs to the scope of protection of the present disclosure.

The blood pressure of the target body is determined on the basis of the pulse wave velocity of the target body, and the accuracy of determining the blood pressure of the target body may be guaranteed.

In the embodiment, a pulse wave velocity measuring device is further provided, and is used for implementing the above embodiments and preferred embodiments, and those that have been illustrated will not be repeated in detail. As used below, the term "module" may be a combination of software and/or hardware which implements a predetermined function. Although the device described in the embodiments below is preferably implemented in software, implementations in hardware, or a combination of software and hardware, are also possible and are conceived.

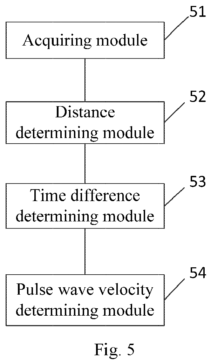

An embodiment provides a pulse wave velocity measuring device, as shown in Fig. 5, comprising:

an acquiring module 51, used for acquiring a target blood vessel ultrasound image of a target body, wherein the target blood vessel ultrasound image comprises at least two sampling areas;

a distance determining module 52, used for acquiring a distance between the at least two sampling areas based on the target blood vessel ultrasound image;

a time difference determining module 53, used for analyzing images in the at least two sampling areas to acquire a time difference of displacement of preset points in a cardiac cycle between the at least two sampling areas; and

a pulse wave velocity determining module 54, used for determining a pulse wave velocity of the target body based on the distance between the at least two sampling areas and the time difference of displacement of the preset points in the cardiac cycle between the at least two sampling areas.

According to the pulse wave velocity measuring device provided by the embodiment, through the at least two sampling areas in the target blood vessel ultrasound image, the distance between the sampling areas and the time difference between the preset points are determined based on the sampling areas in the target blood vessel ultrasound image subsequently, namely, the pulse wave velocity of the target body is quantitatively calculated from the target blood vessel ultrasound image, so that the accuracy of determining the pulse wave velocity of the target body is improved.

The pulse wave velocity measuring device in the embodiment is presented in the form of a functional unit, here is referred to as an ASIC, a processor and a memory executing one or more software or fixed programs, and/or other devices which may provide the above functions.

Further functional description of the above modules is the same as that of the above corresponding embodiment, and the details will not be repeated herein.

An embodiment of the present disclosure also provides an ultrasound device which is provided with the pulse wave velocity measuring device shown in Fig. 5.

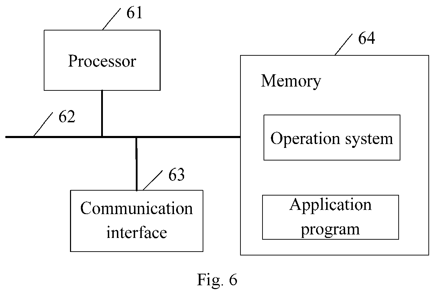

Referring to Fig. 6, Fig. 6 is a structural schematic diagram of an ultrasound device according to an optional embodiment of the present disclosure, as shown in Fig. 6, the ultrasound device may comprise at least one processor 61, such as a CPU (Central Processing Unit), at least one communication interface 63, a memory 64, and at least one communication bus 62. The communication bus 62 is used for implementing connective communications between these components. The communication interface 63 may comprise a display and a keyboard, and the optional communication interface 63 may also comprise a standard wired interface and a wireless interface. The memory 64 may be a high-speed RAM (Random Access Memory) or may also be a non-volatile memory, such as at least one disk memory. The memory 64 may optionally also be at least one storage device away from the foregoing processor 61. The processor 61 may be in combination with a device as described in Fig. 5, an application program is stored in the memory 64, and the processor 61 calls program codes stored in the memory 64 for executing the steps of any one of the above methods.

The communication bus 62 may be a peripheral component interconnect (PCI) bus or an extended industry standard architecture (EISA) bus, and the like. The communication bus 62 may be classified into an address bus, a data bus, a control bus and the like. For facilitating representation, only one thick line is shown in Fig. 6, but it is not shown that there is only one bus or one type of bus.

The memory 64 may comprise a volatile memory, such as a random access memory (RAM); the memory 64 may also comprise a non-volatile memory, such as a flash memory, a hard disk drive (HDD), or a solid-state drive (SSD); and the memory 64 may further comprise a combination of the above types of memories.

The processor 61 may be a central processing unit (CPU), a network processor (NP), or a combination of the CPU and the NP.

The processor 61 may further comprise a hardware chip. The above hardware chip may be an application-specific integrated circuit (ASIC), a programmable logic device (PLD), or a combination thereof. The above PLD may be a complex programmable logic device (CPLD), a field-programmable gate array (FPGA), a generic array logic (GAL), or any combination thereof.

Optionally, the memory 64 is also used for storing program instructions. The processor 61 may call the program instructions to implement the pulse wave velocity measuring method as shown in the embodiments of Fig. 1 to Fig. 4 of the present application.

An embodiment of the present disclosure also provides a non-transitory computer storage medium storing computer-executable instructions for executing the pulse wave velocity measuring method in any of the above method embodiments. The storage medium may be a magnetic disc, an optical disc, a read-only memory (ROM), a random access memory (RAM), a flash memory, a hard disk drive (HDD), or a solid-state drive (SSD); and the storage medium may further comprise a combination of the above types of memories.

Although the embodiments of the present disclosure are described in conjunction with the drawings, those skilled in the art may make various modifications and variations without departing from the spirit and scope of the present disclosure, and these modifications and variations are intended to be within the scope defined by the appended claims.

**Claims**

1. A pulse wave velocity measuring method, comprising:

    acquiring a target blood vessel ultrasound image of a target body, wherein the target blood vessel ultrasound image comprises at least two sampling areas;
    based on the target blood vessel ultrasound image, acquiring a distance between the at least two sampling areas;
    analyzing images in the at least two sampling areas to acquire a time difference of displacement of preset points in a cardiac cycle between the at least two sampling areas; and
    based on the distance between the at least two sampling areas and the time difference of displacement of the preset points in the cardiac cycle between the at least two sampling areas, determining a pulse wave velocity of the target body.

2. The method according to claim 1, wherein there are two sampling areas, and based on the distance between the at least two sampling areas and the time difference of displacement of the preset points in the cardiac cycle between the at least two sampling areas, determining the pulse wave velocity of the target body comprises:

> acquiring a distance between the two sampling areas and a time difference of displacement of preset points in the cardiac cycle between the two sampling areas under preset measurement times;
>
> calculating a ratio of the distance between the two sampling areas to the time difference of displacement of the preset points in the cardiac cycle between the two sampling areas under each measurement to acquire target pulse wave velocities in one-to-one correspondence with the measurement times; and
>
> based on the target pulse wave velocities, determining the pulse wave velocity of the target body.

3. The method according to claim 1, wherein there are at least three sampling areas, and based on the distance between the at least two sampling areas and the time difference of displacement of the preset points in the cardiac cycle between the at least two sampling areas, determining the pulse wave velocity of the target body comprises:

> acquiring a distance and a time difference corresponding to each set of sampling area combination under single measurement, wherein the sampling area combination is a combination of any two sampling areas in the at least three sampling areas;
>
> calculating a ratio of the distance to the time difference corresponding to each set of sampling area combination so as to acquire target pulse wave velocities in one-to-one correspondence to the sampling area combinations; and
>
> based on the target pulse wave velocities, determining the pulse wave velocity of the target body.

4. The method according to claim 2 or 3, wherein based on the target pulse wave velocities, determining the pulse wave velocity of the target body comprises:

> calculating a confidence level of the target pulse wave velocities;
>
> screening the target pulse wave velocities based on the calculated confidence level, and determining the pulse wave velocity of the target body.

5. The method according to claim 1, wherein acquiring the target blood vessel ultrasound image of the target body comprises:

> in response to the setting operation of a working mode, determining the working mode, wherein the working mode comprises a pulse Doppler mode or an M mode;
>
> based on the working mode, acquiring the blood vessel ultrasound image of the target body; and
>
> forming at least two sampling gates or sampling lines on the blood vessel ultrasound image to acquire the target blood vessel ultrasound image.

6. The method according to claim 5, wherein forming the at least two sampling gates or sampling lines on the blood vessel ultrasound image to acquire the target blood vessel ultrasound image comprises:
   in response to the operation of arranging the at least two sampling gates or sampling lines on the blood vessel ultrasound image, forming the at least two sampling gates or sampling lines on the blood vessel ultrasound image to acquire the target blood vessel ultrasound image.

7. The method according to claim 5, wherein forming the at least two sampling gates or sampling lines on the blood vessel ultrasound image to acquire the target blood vessel ultrasound image comprises:

> acquiring at least two preset positions on the blood vessel ultrasound image; and
>
> respectively forming the sampling gates or sampling lines at the at least two preset positions to acquire the target blood vessel ultrasound image.

8. The method according to claim 1, wherein analyzing the images in the at least two sampling areas to acquire the time difference of displacement of the preset points in the cardiac cycle between the at least two sampling areas comprises:

> carrying out binarization processing on the images in the at least two sampling areas to acquire first images corresponding to the sampling areas;
>
> extracting envelope lines in the first images, and determining positions corresponding to the preset points in the cardiac cycle; and
>
> determining the time difference of displacement of the preset points in the cardiac cycle between the at least two sampling areas by utilizing the positions of the preset points.

9. The method according to claim 8, wherein carrying out binarization processing on the images in the at least two sampling areas to acquire the first images corresponding to the sampling areas comprises:

extracting a gray scale map corresponding to the images in the at least two sampling areas; calculating an entropy value corresponding to each gray scale in the gray scale map; determining a gray scale threshold by using the entropy value acquired by calculation; screening pixel points in the gray scale map based on the gray scale threshold to acquire effective pixel points in the gray scale map; and forming the first images by utilizing the effective pixel points.

10. The method according to claim 9, wherein forming the first images by utilizing the effective pixel points comprises:

forming second images by utilizing the effective pixel points; and carrying out corrosion followed by expansion on the second images to acquire the first images.

11. An ultrasound device , comprising: a memory and a processor, the memory and the processor being communicably connected with each other, the memory comprising computer-executable instructions , which when executed by the processor, configure the processor to carry out the pulse wave velocity measuring method of any one of claims 1 to 10.

12. A computer readable storage medium comprising computer-executable instructions, which when executed on a computer configure the computer to carry out the pulse wave velocity measuring method according to any one of claims 1 to 10.

Acquire a target blood vessel ultrasound image of a
target body
/ S11

Based on the target blood vessel ultrasound image,
acquire a distance between at least two sampling
areas
/ S12

Analyze images in the at least two sampling areas to
acquire a time difference of displacement of preset
points in a cardiac cycle between the at least two
sampling areas
/ S13

Based on the distance between the at least two
sampling areas and the time difference of
displacement of the preset points in the cardiac
cycle between the at least two sampling areas,
determine a pulse wave velocity of the target body
/ S14

Fig. 1

Acquire a target blood vessel ultrasound image of a target body — S21

Based on the target blood vessel ultrasound image, acquire a distance between at least two sampling areas — S22

Analyze images in the at least two sampling areas to acquire a time difference of displacement of preset points in a cardiac cycle between the at least two sampling areas — S23

S24

Acquire a distance between two sampling areas and a time difference of displacement of preset points in a cardiac cycle between the two sampling areas under preset measurement times — S241

Calculate a ratio of the distance between the two sampling areas to the time difference of displacement of the preset points in the cardiac cycle between the two sampling areas under each measurement to acquire target pulse wave velocities in one-to-one correspondence with the measurement times — S242

Based on the target pulse wave velocities, determine a pulse wave velocity of the target body — S243

Fig. 2

Fig. 3

Acquire a target blood vessel ultrasound image of a target body — S41

Based on the target blood vessel ultrasound image, acquire a distance between at least two sampling areas — S42

— S43

carry out binarization processing on images in the at least two sampling areas to acquire first images corresponding to the sampling areas — S431

Extract envelope lines in the first images, and determine positions corresponding to preset points in a cardiac cycle — S432

Determine a time difference between the preset points of the at least two sampling areas by utilizing the positions of the preset points — S433

Based on the distance between the at least two sampling areas and the time difference of displacement of the preset points in the cardiac cycle between the at least two sampling areas, determine a pulse wave velocity of a target body — S44

Fig. 4

Fig. 5

Processor 61

62

63
Communication
interface

Memory 64

Operation system

Application
program

Fig. 6

Europäisches
Patentamt

European
Patent Office

Office européen
des brevets

# EUROPEAN SEARCH REPORT

Application Number

EP 21 20 0591

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/081994 A1 (KIM KANG [US] ET AL) 3 April 2008 (2008-04-03) | 1,5,6, 11,12 | INV. A61B8/08 A61B8/06 |
| Y | * abstract * * figures 1-20 * * paragraph [0042] - paragraph [0143] * ----- | 8-10 | |
| X | NABEEL P M ET AL: "Local Pulse Wave Velocity: Theory, Methods, Advancements, and Clinical Applications", IEEE REVIEWS IN BIOMEDICAL ENGINEERING, IEEE, USA, vol. 13, 26 July 2019 (2019-07-26), pages 74-112, XP011766934, ISSN: 1937-3333, DOI: 10.1109/RBME.2019.2931587 [retrieved on 2020-01-17] | 1-3,11, 12 | |
| Y | * abstract * * figures 1,2 * * page 74 - page 104 * ----- | 4 | |
| Y | ZHANG X ET AL: "Measurement of wave velocity in arterial walls with ultrasound transducers", ULTRASOUND IN MEDICINE AND BIOLOGY, NEW YORK, NY, US, vol. 32, no. 11, 2 November 2006 (2006-11-02), pages 1655-1660, XP027881195, ISSN: 0301-5629 [retrieved on 2006-11-01] * abstract * * figures 1-5 * * page 1655 - page 1659 * ----- | 4 | TECHNICAL FIELDS SEARCHED (IPC) A61B |

-/--

The present search report has been drawn up for all claims

| Place of search | Date of completion of the search | Examiner |
|---|---|---|
| Munich | 8 March 2022 | Moehrs, Sascha |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 0 898 938 A2 (TERUMO CORP [JP]) 3 March 1999 (1999-03-03) * abstract * * figures 1-12 * * paragraph [0010] - paragraph [0071] * ----- | 8-10 | |
| A | SORENSEN G L ET AL: "Pulse wave velocity in the carotid artery", ULTRASONICS SYMPOSIUM, 2008. IUS 2008. IEEE, IEEE, PISCATAWAY, NJ, USA, 2 November 2008 (2008-11-02), pages 1386-1389, XP031443480, ISBN: 978-1-4244-2428-3 * abstract * * figures 1-3 * * Sections I - IV * ----- | 1-12 | |

TECHNICAL FIELDS
SEARCHED      (IPC)

The present search report has been drawn up for all claims

| Place of search | Date of completion of the search | Examiner |
|---|---|---|
| Munich | 8 March 2022 | Moehrs, Sascha |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 20 0591

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2022

| Patent document cited in search report | | Publication date | Patent family member(s) | | | Publication date |
|---|---|---|---|---|---|---|
| US 2008081994 | A1 | 03-04-2008 | US | 2008081994 | A1 | 03-04-2008 |
| | | | WO | 2008042409 | A2 | 10-04-2008 |
| EP 0898938 | A2 | 03-03-1999 | DE | 69813970 | T2 | 26-02-2004 |
| | | | EP | 0898938 | A2 | 03-03-1999 |
| | | | JP | 3857788 | B2 | 13-12-2006 |
| | | | JP | H1176233 | A | 23-03-1999 |
| | | | US | 6176832 | B1 | 23-01-2001 |

EPO FORM P0459

For more details about this annex : see Official Journal of the European Patent Office, No. 12/82